# EUROPEAN PATENT APPLICATION

(11) **EP 2 366 380 A1**
(43) Date of publication of application: **21.09.2011**
(21) Application number: 11169586.2
(22) Date of filing: 18.03.2009
(51) Int. Cl.: A61K 9/20, A61K 31/496

(54) **Extended release formulation containing a wax**

(30) Priority: 21.03.2008 US 70332 P
(62) Divisional of application: 09722765.6
(71) Applicant: Mylan Pharmaceuticals, Inc., Morgantown, WV 26505 (US)
(72) Inventor: Chattaraj, Sarat C., Morgantown, WV West Virginia 26505 (US); Bhat, Pavan, Morgantown, WV West Virginia 26505 (US); Redelman, Glenn A., Morgantown, WV West Virginia 26505 (US); Wargo, David J., Pittsburgh, PA Pennsylvania 15228 (US); Addicks, William, Morgantown, WV West Virginia 26505-2900 (US)
(74) Representative: Hamer, Christopher K.

(57) **Abstract**

Extended release pharmaceutical formulations are disclosed wherein the formulations contain an extended release portion and an immediate release portion, the extended release portion comprising an active pharmaceutical ingredient and a wax. Methods of making such extended release pharmaceutical formulations are also disclosed.

## Description

### CROSS-REFERENCE TO RELATED APPLICATIONS

This application claims the benefit of the filing date of United States Provisional Patent Application No. 61/070,332 filed March 21, 2008, the disclosure of which is hereby incorporated herein by reference.

### BACKGROUND OF THE INVENTION

Ciprofloxacin is a broad-spectrum antibiotic that is active against both gram-positive and gram-negative bacteria. The bactericidal action of ciprofloxacin results from inhibition of topoisomerase II (DNA gyrase) and topoisomerase IV, which are required for bacterial DNA replication, transcription, repair and recombination.

Ciprofloxacin tablets were introduced by Bayer Pharmaceuticals under the tradenames Cipro^{®} and Ciproxin^{®}. An intravenous formulation of Cipro^{®} was later introduced in 1991. Cipro^{®} is available in more than 100 countries and has been approved for the treatment of 14 types of infections, especially urinary tract infections such as acute uncomplicated cystitis, pyelonephritis, and chronic bacterial prostatitis.

An extended release version of ciprofloxacin was approved by the FDA on December 13, 2002 and marketed by Bayer Pharmaceuticals under the tradename Cipro XR^{®}. Cipro XR^{®} tablets are coated, bi-layer tablets consisting of an immediate-release layer and an erosion-matrix type controlled-release layer. Cipro XR^{®} tablets are formulated to release at least some of the active pharmaceutical ingredient over an extended period of time. Approximately 35% of the dose is contained within the immediate-release component, while the remaining 65% is contained in a slow release matrix.

The tablets produced by Bayer contain a combination of two types of ciprofloxacin drug substance, ciprofloxacin hydrochloride and ciprofloxacin betaine (base). Cipro XR^{®} is available as a 500mg or 1000mg (ciprofloxacin equivalent) tablet strength. The inactive ingredients are crospovidone, hypromellose, magnesium stearate, polyethylene glycol, silica colloidal anhydrate, succinic acid, and titanium dioxide. Cipro XR^{®} tablets are nearly white to slightly yellowish film coated tablet having an oblong shape.

Maximum plasma ciprofloxacin concentrations are obtained between about 1 and about 4 hours after dosing with Cipro XR^{®}. In comparison to the 250mg ciprofloxacin immediate-release treatments, which are approved for treatment of uncomplicated urinary tract infections, the Cₘₐₓ of Cipro XR^{®} 500mg once daily is higher, while the area under the curve over 24 hours is equivalent. Moreover, the results of the pharmacokinetic studies demonstrate that Cipro XR^{®} may be administered with or without food (for example high-fat and low-fat meals are under fasted conditions).

United States Patent Publication No. 2006/0275367 describes extended release formulations of poorly water soluble drugs.

United States Patent No. 6,039,974 describes a bilayer tablet composition comprising, in a first layer, a decongestant, carnauba wax, and an anti-adherent; and an antihistamine in a second layer. The patent discloses a high amount of carnauba in the first layer (59% to 81%) and a comparatively low amount of API.

There remains a need to produce extended release formulations of APIs that are bioequivalent to commercially available dosage forms or USP dosage forms, such as CiproXR^{®}.

### SUMMARY OF THE INVENTION

In accordance with the present invention, an extended release formulation has been discovered comprising an extended release portion and an immediate release portion, wherein said extended release portion comprises an active pharmaceutical ingredient, and/or the salt, solvate, or hydrate thereof (hereinafter "API") and at least one wax-based or waxy material (hereinafter "wax"). The term "wax" is intended to mean fatty acids, fatty acid ester derivatives, higher alcohols, higher alcohol ester derivatives, and others described herein.

In accordance with another embodiment of the present invention, the wax is selected from the group consisting of higher fatty acids, higher fatty acid ester derivatives, higher alcohols, and higher alcohol derivatives. In accordance with another embodiment of the present invention, the wax is selected from the group consisting of carnauba wax, white wax, bees wax, glycerol monostearate, glycerol oleate, paraffin, and spermaceti.

In accordance with another embodiment, the amount of wax in the extended release portion ranges from about 2% w/w to about 40% w/w by weight of the extended release portion. In accordance with another embodiment of the present invention, the amount of wax in the extended release portion ranges from about 4% w/w to about 30% w/w by weight of the extended release portion. In accordance with another embodiment of the present invention, the amount of wax in the extended release portion ranges from about 6% w/w to about 23% w/w by weight of the extended release portion.

In accordance with another embodiment of the present invention, the API is a water soluble free base and/or salt (including the soluble salt of an insoluble free base). In accordance with another embodiment of the present invention, the API in the extended release portion is selected from the group consisting of propranolol, metoprolol tartrate, metoprolol succinate, galantamine, bupropion, diltiazem, oxybutynin, hydrochlorothiazide, metformin, opamine, ciprofloxacin, vancomycin, norvancomycin, daunorubicin, vinca alkaloids, cetrizine, venlafaxine, opioid analgesics, theophylline, verapamil, amlodipine, atomoxetine, zopiclone, tramadol, timolol, trospium, pramipexole, and pharmaceutically acceptable salts, hydrates, or solvates thereof.

In accordance with another embodiment of the present invention, an amount of said API in the extended release portion ranges from about 5% w/w to about 75% w/w by weight of the extended release portion. In accordance with another embodiment of the present invention, the extended release portion contains a mixture of a free base and a salt of the API.

In accordance with another embodiment of the present invention, the extended release portion further comprises an additional matrix-forming component. The matrix-forming component may be selected, for example, from capric acid, lauric acid, myristic acid, palmitic acid, stearic acid, arachidonic acid, and behenic acid. In accordance with another embodiment of the present invention, the matrix-forming component is selected from the group consisting of succinic acid, citric acid, malic acid, lactic acid, aspartic acid, glutamic acid, gluconic acid, acetic acid, formic acid, hydrochloric acid, sulphuric acid, phosphoric acid, hydrophilic polymers, polyethylene glycols, pH dependent acrylate polymers or copolymers, and pore forming agents.

In accordance with another embodiment of the present invention, the extended release portion comprises one or more additives. In one embodiment, dibasic calcium phosphate (which may be selected from, for example, the dihydrate or anhydrous forms) is used as an insoluble filler for the matrix of the extended release portion of the formulation. The amount of dibasic calcium phosphate generally ranges between about 3% w/w to about 30% w/w by weight of the extended release portion. In another embodiment, starch or microcrystalline cellulose is used as an insoluble filler.

In accordance with another embodiment of the present invention, the immediate release portion comprises an API, which may be the same or different than the API in the extended release portion. The API may be a free base, salt, solvate, or hydrate, or mixtures thereof. In another embodiment, the API is present as a mixture of the free base and salt. In accordance with another embodiment of the present invention, an amount of the API in the immediate release portion ranges from about 5% w/w to about 80% w/w by weight of the immediate release portion.

In accordance with another embodiment of the present invention, the extended release and immediate release portions are combined in a tablet, bilayer tablet, a capsule, or any other suitable dosage form (hereinafter "dosage form"). In accordance with another embodiment of the present invention, an amount of the extended release portion in the dosage form ranges from about 9:1 to about 1:9.

In accordance with another embodiment of the present invention, about 5% to about 75% of the API in the formulation is released after about 1 hour, and about 25% to about 100% of the API in the formulation is released after about 6 hours.

In accordance with another embodiment of the present invention, a ratio of a peak concentration for a fasted condition to a peak concentration for a fed condition ranges from about 0.8 to about 1.2.

In accordance with another embodiment of the present invention, a ratio of an area under a curve for a fasted condition to an area under a curve for a fed condition ranges from about 0.8 to about 1.2.

In another aspect of the present invention is an extended release pharmaceutical formulation comprising an extended release portion and an immediate release portion, wherein the extended release portion comprises ciprofloxacin its salt, solvate, or hydrate or a mixture thereof (hereinafter referred to as "ciprofloxacin") and at least one wax. In accordance with another embodiment of the present invention, the wax is carnauba wax. The extended release portion may further comprise an additional matrix forming component. In accordance with another embodiment of the present invention, the immediate release portion also comprises ciprofloxacin its salt, solvate, or hydrate or a mixture thereof. In accordance with another embodiment of the present invention, the immediate release portion is selected from an API different from that in the extended release portion, i.e. the API is different than ciprofloxacin. In accordance with another embodiment of the present invention, the extended release portion further comprises stearic acid.

In another aspect of the present invention is a pharmaceutical formulation comprising extended and immediate release portions, the extended release portion comprising an API and a wax, the amount of wax is less than about 50% by weight of the extended release portion. In one embodiment, the API in the extended release portion is ciprofloxacin. The API in the immediate release portion may be the same or different.

In yet another aspect of the present invention is a method of forming an extended release portion comprising an API and a wax and, an immediate release portion, and combining the two portions within a suitable dosage form.

In yet a further aspect of the present invention is a method of treating a subject comprising administering an extended release formulation comprising an extended release portion and an immediate release portion, wherein the extended release portion is comprised of an API and at least one wax.

In another embodiment, is a method of treating a bacterial infection comprising administering a formulation comprising extended and immediate release portions, the extended release portion comprising ciprofloxacin and a wax, the amount of wax ranging from about 2% to about 40% by weight of the extended release portion. For example, the bacterial infection may be a respiratory infection or urinary infection.

It has been discovered that the formulations of the present invention provide for extended release of one or more APIs from a bilayer tablet and can be employed for even poorly water soluble APIs, such as ciprofloxacin free base. It is believed that using a wax in an amount of less than 50% by weight of the extended release portion provides for an erodable matrix from which a sufficient amount of API is released over time.

### DETAILED DESCRIPTION

In one aspect of the present invention is an extended release pharmaceutical formulation (hereinafter "formulation") comprising an extended release portion and an immediate release portion, wherein the extended release portion comprises an active pharmaceutical ingredient (API) its salt, solvate, or hydrate thereof or mixtures thereof and at least one wax.

The extended release formulation is provided in a suitable dosage form. One skilled in the art will be able to construct an appropriate dosage form containing both the extended release and immediate release portions of the present invention. If the dosage form is a bilayer tablet, then at least part of one side of the bilayer tablet contains the extended release portion and at least part of the opposite side of the bilayer tablet contains the immediate release portion.

Each of the extended release and immediate release portions, as well as the final dosage form containing the formulation, will be discussed in more detail below.

Extended Release Portion

The term "extended release" refers to the fact that the API is released from the extended release portion at a rate such that therapeutically beneficial blood levels of the API are maintained over a prolonged period of time, e.g. the API is released from the extended release portion for a time period ranging from about 30 minutes to about 12 hours after administration, preferably for a time period ranging from about 30 minutes to about 8 hours after administration.

The extended release portion of the present formulation comprises an API and a wax. It is believed that the wax, and any associated matrix-forming components as described below, provides a matrix from which the API is released over an extended period of time.

The API may be selected from water soluble and insoluble active pharmaceutical ingredients (including the salts, solvates, and hydrates of the API). One skilled in the art will realize that an otherwise insoluble API may be soluble when in the form of a salt. By way of example only, ciprofloxacin is more soluble as a salt than the free base alone.

In general, the active pharmaceutical ingredients may be selected from the groups consisting of antiinflammatory substances, coronary dilators, cerebal vaso dilators, peripheral vasodilators, anti-infectives, psychotropics, anti-maniics, stimulants, gastro-intestinal sedatives, anti-anginal drugs, vasodilators, anti-arrhythmics, anti-hypertensive drugs, vasoconstrictors, migraine treatments, anti-coagulants and anti-thrombotic drugs, analgesics, anti-pyretics, hypnotics, sedatives, anti-emetics, anti-nauseants, anti-convulsants, neuromuscular drugs, hyper-and hypoglycemic agents, thyroid and anti-thyroid preparations, diuretics, anti-spasmodics, uterine relaxants, anti-obesity drugs, anabolic drugs, erythropoietic drugs, anti-asthmatics, bronchodilators, expectorants, cough suppressants, mucolytics anti-uricemic drugs and the like.

In some embodiments, the active pharmaceutical ingredient is water soluble, having a solubility greater than 1 part solute to 30 parts solvent. Water soluble API's include the salts and solvates of APIs formed with inorganic and/or organic acids that are positively charged due to non-covalently attached protons, permanently positively (or negatively) charged molecules, and negatively charged molecules that are salts of weak and strong acids. In other embodiments, the API (or salt, solvate, of hydrate thereof) is freely soluble, having a solubility of about 1 part solute to about 10 parts solvent. In yet other embodiments, the API (or salt, solvate, or hydrate) is soluble, having a solubility of about 1 part solute to about 1 part solvent or less.

Specific APIs may be selected from the group consisting of propranolol, metoprolol tartrate, metoprolol succinate, galantamine, bupropion, diltiazem, oxybutynin, hydrochlorothiazide, metformin, ciprofloxacin, vancomycin, norvancomycin, daunorubicin, vinca alkaloids (e.g., vinorelbine), venlafaxine, opioid analgesics (e.g., morphine), theophylline, verapamil, amlodipine, tramadol, diltiazem, timolol, trospium, pramipexole, enalapril, and pharmaceutically acceptable salts, hydrates, or solvates thereof. Other APIs include, azithromycin, clarithromycin, erythromycin, cefalaxin, doxycycline, atenolol, divalproex sodium, niacin, darifenacin, telithromycin, minocycline hydrochloride, amoxicillin, clavulanate potassium, dexmethylphenidate hydrochloride, bupropion hydrochloride, rosiglitazone, glimepiride, olmesartan medoximil, hydrochlorothiazide, carbidopa, levodopa, emtricitabine, zidovudin, abacavir, lamivudine, lopinavir, ritonavir, losartan, tramadol, olanzapine, oxycodone, trandolapril, atorvastatin, nifedipine, rosuvastatin, simvastatin, lovastatin, and pharmaceutically acceptable salts, hydrates, or solvates thereof.

The API in the extended release portion may be present as a free base or as the salt, solvate, hydrate thereof. In some embodiments, the API is a salt. In other embodiments, the API is the hydrochloride salt, the sulfuric acid salt, the citrate salt, the malic acid salt, the stearate salt, the phosphate salt, the succinate salt, the lactic acid salt, the aspartic acid salt, the glutamic acid salt, the gluconic acid salt, the acetate salt, the besylate salt, or the formate salt. In yet other embodiments, the API may be present in a mixture of two forms, such as, for example, the free base and a salt, or a mixture of two different salt forms.

Without wishing to be bound by any one particular theory, it is believed that by combining a salt and a free base allows for one skilled in the art to control the release of a particular API since the salt and free base forms each may have different solubility rates. It is also believed that such a combination may actually assist in providing an extended release of API over time.

In one embodiment, the extended release portion contains the free base of an API and the hydrochloride salt of the same API. In another embodiment the API is ciprofloxacin and/or a salt, solvate, or hydrate thereof. In one embodiment the API is ciprofloxacin.

The amount of API in the extended release portion ranges from between about 5% w/w and about 75% w/w by weight of the extended release portion, preferably from about 30% w/w to about 70% w/w by weight of the extended release portion, and more preferably from about 55% w/w to about 65% w/w by weight of the extended release portion.

In the embodiments where the extended release portion comprises the free base of an API and the salt of the same API, the amount of free base to salt ranges from about 1:2 to about 2:1.

The wax provides the desired extended release profile for the formulation by creating a matrix, alone or in combination with another matrix-forming component, from which the API is released. Any wax may be used in the extended release portion provided that it is safe for oral pharmaceutical formulations and will not interfere with the mechanism of action of the API. Suitable waxes include, but are not limited to, waxes of animal or vegetable origin, synthetic waxes, and semi-synthetic waxes. In particular, the waxes include higher fatty acids, higher fatty acid ester derivatives, higher alcohols, and higher alcohol ester derivatives. For example, the wax may be an ester of a high molecular weight monohydric alcohol or a high molecular weight fatty acid.

Higher fatty acids include, without limitation, lauric acid, tridecanoic acid, myristic acid, pentadecanoic acid, palmitic acid, margaric acid, stearic acid, nonadecanoic acid, arachidonic acid, behenic acid, lignoceric acid, cerotic acid, montanic acid, and mixtures thereof.

Higher fatty acid ester derivatives include, without limitation, glyceryl, ethylene glycol, propylene glycol, sorbitol, polyethylene glycol and other esters of the higher fatty acids listed above; saturated fatty acid glycerides derived from animals or vegetable, mixtures thereof, and hydrogenated oils available from said glycerides of the animal or vegitable origin; glycerides of oleic acid, linolic acid, linolenic acid, and ricinoleic acid.

Higher alcohols include, without limitation, pentadecanol, hexadecanol, heptadecanol, octadecanol, nonadecanol, eicosanol, wool alcohol, and cholesterol.

Higher alcohol ester derivatives include, without limitation, cholesteryl palmitate and phytosterol palmitate.

In some embodiments, the wax is a high melting (e.g. melts above 35°C), pharmaceutically acceptable water-insoluble wax, such as a saturated fat. Examples of high melting, water-insoluble waxes include, without limitation, carnauba wax, white wax, bees wax, glycerol monostearate, glycerol oleate, paraffin, or spermaceti. In preferred embodiments, the wax is carnauba wax or a mixture or derivative thereof.

Generally, less than about 50% of the extended portion is comprised of wax. The amount of wax in the extended release portion ranges from about 2% w/w to about 40% w/w by weight of the extended release portion, preferably from about 4% w/w to about 30% w/w by weight of the extended release portion, more preferably from about 6% w/w to about 23% w/w by weight of the extended release portion, and yet more preferably from about 7% w/w to about 11% w/w by weight of the extended release portion.

Those skilled in the art will recognize that the release of API from the extended-release portion can be varied by changing the amounts or type of wax in the extended release portion, by combining two or more waxes in varying amounts, or by combining one or more waxes with another matrix-forming component that could modulate API release, such as, for example, a polymer, a copolymer, a channeling agent, a carbohydrate, an inorganic acid, or an organic acid.

Suitable soluble matrix-forming components which may be combined with the wax include succinic acid, citric acid, malic acid, stearic acid, lactic acid, aspartic acid, glutamic acid, gluconic acid, acetic acid, formic acid, hydrochloric acid, sulphuric acid, and phosphoric acid. Other suitable matrix-forming components include hydrophilic polymers such as hypromellose, hydroxypropylcellulose, polyvinylpyrrolidone, hydrophilic cellulose, polyethylene oxide and polyethylene glycols. Yet other suitable matrix-forming components include pH dependent acrylate polymers and copolymers (including, but not limited to, Eudragit L100-55, Eudragit S100, and Eudragit L100) from Rohm and Haas and other traditional pore forming agents. Pore forming agents include, without limitation, lactose, mannitol, sucrose, dextrose, fructose, xylitol, sorbitol, and electrolytes (e.g. sodium chloride, potassium chloride, etc.). In some embodiments, the wax is combined with stearic acid to form a matrix.

If the wax is combined with an additional matrix-forming component, the amount of additional matrix-forming component ranges from about 1% to about 15% by weight of the extended release portion, preferably from about 2% to about 8% by weight of the extended release portion.

The extended release portion may also be combined with other additives and/or excipients (collectively "additives") to yield an appropriate release of API from the extended release portion or to aid in the tableting process. Suitable additives include acidifiers, binders, fillers, osmotic agents, diluents, absorbents, colorants, dyes, pigments, disintegrants, dispersants, encapsulants, flow aids, hardeners, permeation enhancers, demulcents, stabilizers, tableting aids, glidants, lubricants, plasticizers, stabilizers, anti-tacking agents, and wetting agents. Any additive utilized must be pharmaceutically acceptable and compatible with the API, the wax, and the other matrix-forming component (s) . Moreover, within these parameters, any combination of additives may be utilized in the extended release portion of the present invention.

The additive(s) may be added by methods known in the art, for example, by directly mixing with the wax-based extended release material and the active ingredient or adding the additive while mixing the granulate containing the wax-based extended release material and the active ingredient.

Suitable binders include polyvinyl-pyrrolidine, hydroxypropyl cellulose, methylcellulose, hydroxypropyl methylcellulose, sugars (e.g., glucose), acacia, carboxymethylcellulose sodium, dextrin, ethylcellulose, gelatin, pregelatinized starch, sodium alginate, zein, polyvinypyrrolidone, carbomer, acrylic acid polymers, polyethylene oxide and the like or mixtures thereof.

Suitable disintegrants include alginic acid, crosscarmellose, crospovidone, low-substituted hydroxypropyl cellulose, polacrilin potassium and sodium starch glycolate.

Suitable lubricants include talc, magnesium stearate, stearic acid calcium stearate, hydrogenated vegetable oil sodium benzoate, and sodium stearyl fumarate.

Suitable fillers include carboxymethylcellulose, ascorbic acid, glutamic acid, phosphoric acid, sorbic acid, tartaric acid, triethyl-citrate, electrolytes, sucrose, mannitol, sorbitol, dextrose, lactose, microcrystalline cellulose, fructose, xylitol, starches, and the like or mixtures thereof.

Suitable acidifiers include organic and inorganic acids such as tartaric acid, citric acid, hydrochloric acid, sulphuric acid, ascorbic acid, isoascorbic acid, cysteine hydrochloride, and glycine hydrochloride.

Suitable wetting agents include polyethylene glycols and their esters or ethers, anionic surfactants such as sodium lauryl sulfate, sodium, potassium or magnesium n-dodecyl sulfate, n-tetradecylsulfate, n-hexadecyl sulfate, n-tetradecyloxyethyl sulfate, n-hexadecyloxyethyl sulfate or n-octadecyloxyethyl sulfate, sodium, potassium or magnesium n-dodecanesulfonate, sodium, potassium or magnesium n-tetradecanesulfonate, n-hexadecanesulfonate or n-octadecanesulfonate, and the like.

In some embodiments, the extended release portion may comprises an API, a wax, another matrix-forming component, a filler, a disintegrant, and an acidifier. In other embodiments, the API and wax is combined with stearic acid, microcrystalline cellulose, starch, calcium phosphate, and succinic acid. In yet other embodiments, the API and wax is combined with stearic acid, microcrystalline cellulose, starch, calcium phosphate, succinic acid, and a lubricant, such as magnesium stearate.

In one embodiment, dibasic calcium phosphate (which may be selected from, for example, the dihydrate or anhydrous forms) is used as an insoluble filler for the matrix of the extended release portion of the formulation. The amount of dibasic calcium phosphate may range between about 3% w/w to about 30% w/w by weight of the extended release portion. In another embodiment, starch or microcrystalline cellulose was used as an insoluble filler.

Generally, the amount of additive in the extended release portion ranges from about 8% w/w to about 80% w/w by weight of the extended release portion, preferably from about 9% w/w to about 60% w/w by weight of the extended release portion, most preferably from about 12% w/w to about 40% w/w by weight of the extended release portion.

Immediate Release Portion

The extended release portion is combined with an immediate release portion such that therapeutically beneficial blood levels of the API can be achieved upon oral administration, or shortly thereafter. The term "immediate release" refers to the fact that the release of an API is not significantly delayed from the formulation by, for example, means of a protective coating or by embedding the API in a matrix. The immediate release portion is designed to at least partially release API within about 3 minutes to about 60 minutes after administration of the formulation, preferably the immediate release portion is designed to at least partially release API within about 5 minutes to about 35 minutes after administration of the formulation.

The immediate release portion of the extended release formulation comprises an API, and optionally one or more additives. Any API may be used, including, but not limited to, those enumerated above.

As with the extended release portion, the API in the immediate release portion may be present as a free base or as the salt, solvate, or hydrate or mixtures thereof. In some embodiments, the API is a salt. In other embodiments, the API is the hydrochloride salt, the sulfuric acid salt, the citrate salt, the malic acid salt, the stearate salt, the phosphate salt, the succinate salt, the lactic acid salt, the aspartic acid salt, the glutamic acid salt, the gluconic acid salt, the acetate salt, or the formate salt.

The same API or different APIs may be used in the extended release and immediate release portions. For example, amlodipine may be used in an immediate release portion while benazepril may be used in an extended release portion of a bilayer tablet. Other non-limiting examples where different APIs (in any arrangement) are used in the extended release and immediate release portions include combinations such as amlodipine/atorvastatin, pioglitazone/metformin, glyburide/metformin, olmesartan medoxomil/ hydrochlorothiazide, amlodipine/valsartan, misoprostol/diclofenac sodium, misoprostol/piroxicam, trandolapril/verapamil hydrochloride, lisinopril/hydrochlorothiazide, and glyburide/metformin. Yet other non-limiting examples where different APIs (in any arrangement) are used in the extended release and immediate release portions include combinations such as amoxicillin/clavulanate potassium, dexmethylphenidate hydrochloride, bupropion hydrochloride, rosiglitazone/glimepiride, olmesartan medoximil/hydrochlorothiazide, candesartan cilexetil/hydrochlorothiazide, carbidopa/levodopa, emtricitabine/tenofovir disoproxil fumarate/efavirenz, lamivudin/zidovudin, abacavir/lamivudin, lopinavir/ritonavir, losartan/hydrochlorothiazide, tramadol/acetamenophen, olanzapine/fluoxetine hydrochloride, oxycodone/ acetamenophen, trandolapril/verapamil, atorvastatin/nifedipine, atorvastatin/niacin, rosuvastatin/niacin, simvastatin/ezetimibe, and lovastatin/niacin. The different APIs used in each portion can be independently present as a free base, salt, solvate, or hydrate. Of course, there are no restrictions on which API can be included in any portion.

One skilled in the art will recognize that if the same API is used in the extended release and the immediate release portions, different salts, solvates, or hydrates of the API may be used in each portion. For example, the hydrochloride salt of an API may be used in one portion while the citrate salt of the same API is used in the other portion. Similarly, the free base of an API may be used in one portion while the salt, solvate, or hydrate of the same API is used in the other portion.

The API in the immediate release portion may be present as a mixture of two forms, such as, for example, the free base and the salt, regardless of whether the API in the immediate release portion is the same as the API in the extended release portion. In some embodiments, the immediate release portion contains the free base of an API and the salt of the API. In other embodiments, the immediate release portion contains the free base of an API and the hydrochloride salt of the API. In yet other embodiments the API is ciprofloxacin. In yet further embodiments, ciprofloxacin is in both the extended release and immediate release portions.

The amount of API in the immediate release portion ranges from between about 5% w/w and about 80% w/w by weight of the immediate release portion, preferably from about 55% w/w to about 75% w/w by weight of the immediate release portion, and more preferably from about 45% w/w to about 70% w/w by weight of the immediate release portion.

When the immediate release portion contains the free base of an API and the salt of the same API, the amount of free base to salt ranges from about 0.1:1 to about 1:0.1, preferably from about 0.4:1 to about 1:0.4.

The immediate release portion may also be combined with additives, such as those disclosed above, to yield an appropriate release of the API from the immediate release portion or to aid in the tableting process.

In some embodiments, the API in the immediate release portion is combined with a binder, a flow aid, a disintegrant, and a glidant. In other embodiments, the API in the immediate release portion is combined with microcrystalline cellulose, polyvinypyrrolidone, croscarmellose sodium, and colloidal silicone dioxide, such as Cab-O-Sil^{®} manufactured by Cabot Corporation.

Generally, the amount of additive in the immediate release portion ranges from about 10% to about 90% by weight of the immediate release portion, preferably from about 15% to about 70% by weight of the immediate release portion, more preferably from about 15% to about 40% by weight of the immediate release portion.

Final Formulation

The extended release and immediate release portions are combined into a tablet, bilayer tablet, or capsule. In bilayer tablet embodiments, the extended release portion occupies at least a portion of one side of the tablet and the immediate release portion occupies at least a portion of the opposite side of the tablet. Indeed, any one portion does not have to occupy an entire side of the bilayer tablet. One of skill in the art will be able to design an appropriately shaped bilayer tablet (e.g. round or oval) to optimize API release.

The dosage form may also contain other additives (such as those described above) to further enhance drug release, aid in the tableting process, or to increase the bulk of the tablet. It is within the purview of one of ordinary skill in the art to determine how much additive is to be included and the objective that one wishes to accomplish by adding the same. Other pharmaceutically acceptable ingredients which may be added include coloring agents, preservatives, artificial sweeteners, flavorants, antioxidants, and the like. For example, the dosage form may be coated with any traditional coatings to prevent sticking of the tablets, or to enable printing on the tablets. Moreover, the dosage form can be coated with taste masking agents or other biodegradable polymers to make it easier to swallow.

In some embodiments, the amount of extended release portion to immediate release portion in the dosage form ranges from about 9:1 to about 1:9. In other embodiments, the amount of extended release portion to immediate release portion in the dosage form ranges from about 7:1 to about 1:7. In yet other embodiments, the amount of extended release portion to immediate release portion in the dosage form ranges from about 5:1 to about 1:5.

The total amount of API(s) or the amounts of any one portion in the final formulation may vary depending on the desired dose. The total amount of API(s) used is dependent on the patient's age, weight, sex, medical condition, disease or any other medical criteria. The extended release formulations of the present invention may be administered one or more times per day, as determined by the patient's medical care provider. The dosage form of the invention is suitable for twice-a-day administration.

In some embodiments of the present invention, the extended release formulations provide a dissolution profile in aqueous media such that about 5% to about 75% of the API is released after about 1 hour; and about 25% to about 100% of the API is released after about 6 hours. The dissolution media and apparatus was as follows: 900mL, 0.1 N HCl @ 37°C +/- 0.5°C, 2 paddle @50 rpm.

In other embodiments, the extended release formulations provide a dissolution profile in aqueous media such that about 0% to about 20% of the API is released from the extended release formulation after about 0.25 hours; about 15% to about 35% of the API is released after about 0.5 hours; about 35% to about 50% of the API is released after 1 hour; about 50% to about 75% of the API is released after about 2 hours; about 60% to about 85% of the API is released after about 6 hours; and more than 80% is released after about 8 hours.

In yet other embodiments, the extended release formulations provide a dissolution profile in aqueous media such that about 0.25% to about 30% of the API is released from the extended release formulation after about 0.25 hours; about 20% to about 60% of the API is released after about 0.5 hours; about 35% to about 70% of the API is released after about 0.75 hours; about 50% to about 85% of the API is released after about 1 hour; and about 65% to about 100% of the API is released after about 6 hours.

Studies were performed to determine the effect of food on the dosing of the pharmaceutical formulations of the present invention. Area under the curve ("AUCL") was measured for both fed and fasted conditions. AUCL refers to the area under the total API plasma concentration-time curve from time zero to the last quantifiable concentration. In some embodiments, the ratio of an area under the curve for fasted conditions to an area under the curve for fed conditions for the extended release formulations of the present invention ranges from about 0.8 to about 1.2. In other embodiments, this ratio ranges from about 0.9 to about 1.1.

Peak concentration ("CPEAK") was also measured for both fed and fasted conditions. CPEAK refers to the maximum drug concentration obtained directly from the data without interpolation. In some embodiments, the ratio of a peak concentration for fasted conditions to a peak concentration for fed conditions for the extended release pharmaceutical formulations of the present invention ranges from about 0.8 to about 1.2. In other embodiments, this ratio ranges from about 0.9 to 1.1.

Method of Forming the Extended Release Formulation

Also disclosed are methods of preparing the extended release and immediate release portions, as well as the dosage form. The extended release and immediate release portions may be prepared by the same or different methods. These methods are generally known to those of skill in the art.

Generally, the extended release portion is prepared by granulation, preferably by hot melt granulation. The ingredients can be added in any order during the granulation process. Granulation can take place in any conventional manner to produce a blend. For example, it can be prepared using a jacketed bowl equipped with a planetary mixer, or using a hot melt extruder or a fluid bed granulator, coated and mixed in a twin shell blender, a V-shaped blender, a double cone blender, a ribbon mixer, and the like.

In one embodiment, the wax (and any optional matrix-forming components) is added to a jacketed mixing tank equipped with a vertical clamp mount mixer. The materials are heated to a temperature dependent upon the type of wax and other components. In some embodiments, the component(s) are heated to temperature between 80°C and 165°C. The material(s) were mixed to produce a molten solution.

The API is added to, mixed, and granulated with the molten solution. The extended release portion granulation is then cooled, or allowed to cool, to produce an extended release cooled material.

The extended release cooled material is then milled to produce the extended release portion milled material. At this point, additives are blended with the milled material, discharged, and then screened, to produce the extended release formulation blend. These may be further processed to form particles, beads, microcapsules, pellets, etc.

Generally, the immediate release portion is produced by granulation, preferably by wet granulation. First, the granulating suspension is produced. Additives, such as polyvinypyrrolidone, can be added to the granulating suspension. The API and any additives are added to a bowl, mixed, and granulated with the granulating suspension. The immediate release granulation is then dried using a fluid bed drier, milled, combined/blended with any additives, discharged, and then screened. These may be further processed to form particles, beads, microcapsules, pellets, etc.

The extended and immediate release portions, once prepared by their respective granulation processes, have about the following particle size distributions:

| | Extended- release Portion | Immediate- release Portion |
|---|---|---|
| Sieve Size (Mesh) | %Retain on | %Retain on |
| #20 | 1 | 1 |
| #40 | 32 | 25 |
| #60 | 19 | 17 |
| #80 | 11 | 9 |
| #100 | 5 | 4 |
| #140 | 9 | 10 |
| Pan | 23 | 34 |

In some embodiments, a majority of the particles in the extended release portion have a size of at least about 40 mesh. In other embodiments, a majority of the particles in the immediate release portion have a size of at least about 40 mesh. In yet other embodiments, the majority of the total particles in the extended and immediate release portions have a size ranging from about 40 mesh to about 140 mesh.

To produce a bilayer tablet, the extended and immediate release portions are compressed, for example, on a rotary bi-layer tablet press. Any additional additives or lubricants can be added at this time. The compressed tablets are then coated with one or more coating solutions. For example, a coating solution can be comprised of Clear Opadry^{®}, available from Colorcon^{®}, and applied to the bilayer tablet.

Alternatively, suitable amounts of extended and immediate release portions (granules, particles, beads, pellets, microcapsules, etc.) can be combined in a capsule as known to those skilled in the art.

Examples

Examples 1-4:

Table 1 shows ciprofloxacin bilayer tablets, each of examples 1-4 contain an extended release portion and an immediate release portion.

**Table 1:**

| | Example 1 | | Example 2 | | Example 3 | | Example 4 | |
|---|---|---|---|---|---|---|---|---|
| Extended Release ("ER") Portion | (65.3%/34.7%) | | (66.5%/33.5%) | | (66.5%/33.5%) | | (66.5%/33.5%) | |
| | mg | % | mg | % | mg | % | mg | % |
| Carnauba Wax | 52.9 | 9.5 | 53.8 | 9.2 | 53.8 | 9.2 | 52.9 | 9.0 |
| Stearic Acid | 17.6 | 3.2 | 18.0 | 3.1 | 18.0 | 3.1 | 17.6 | 3.0 |
| Ciprofloxacin HCl | 218.6 | 39.4 | 340.9 | 58.3 | 147.4 | 25.2 | 218.6 | 37.4 |
| Ciprofloxacin Base | 138.8 | 25.0 | 23.0 | 3.9 | 216.5 | 37.0 | 138.8 | 23.7 |
| Succinic Acid | - | - | 20 | 3.4 | 20 | 3.4 | 30 | 5.1 |
| Microcrystalline Cellulose | 31.6 | 5.7 | 32.2 | 5.5 | 32.2 | 5.5 | 31.6 | 5.4 |
| Dibasic Calcium Phosphate | 31.6 | 5.7 | 32.2 | 5.5 | 32.2 | 5.5 | 31.6 | 5.4 |
| Pregelatinized Starch | 55.5 | 10.0 | 56.5 | 9.6 | 56.5 | 9.6 | 55.5 | 9.4 |
| colloidal silicone dioxide | 2.8 | 0.5 | 2.9 | 0.5 | 2.9 | 0.5 | 2.8 | 0.5 |
| Magnesium Stearate/Sodium Lauryl Sulfate (94/6) | 5.55 | 1.0 | 5.7 | 1.0 | 5.7 | 1.0 | 5.55 | 1.0 |
| ER Layer Weight | 555 | | 585 | | 585 | | 585 | |
| Immediate Release ("IR") Portion | | | | | | | | |
| Polyvinylpyrrolidone | 5.9 | 2.0 | 5.7 | 1.9 | 5.7 | 1.9 | 5.9 | 2.0 |
| Ciprofloxacin HCl, USP | 116.2 | 45.2 | - | - | 183.4 | 62.2 | 116.2 | 45.2 |
| Ciprofloxacin Base | 73.8 | 28.7 | 183.4 | 62.2 | - | - | 73.8 | 28.7 |
| Microcrystalline Cellulose | 82.9 | 28.1 | 90.1 | 30.5 | 90.1 | 30.5 | 82.9 | 28.1 |
| Croscarmellose Sodium | 11.8 | 4.0 | 11.4 | 3.9 | 11.4 | 3.9 | 11.8 | 4.0 |
| colloidal silicone dioxide | 1.47 | 0.5 | 1.42 | 0.5 | 1.42 | 0.5 | 1.48 | 0.5 |
| Magnesium Stearate/Sodium Lauryl Sulfate (94/6) | 2.95 | 1.0 | 2.85 | 1.0 | 2.85 | 1.0 | 2.95 | 1.0 |
| IR Layer Weight | 295 | | 295 | | 295 | | 295 | |
| Total Tablet Weight, mg | 850 | | 880 | | 880 | | 880 | |

Examples 1 to 4 each include an extended release portion and an immediate release portion in a bilayer tablet. The extended release portion in each of these examples comprises a mixture of the free base and hydrochloride salt of ciprofloxacin. The API is released from a matrix of carnauba wax and stearic acid. Other additives are present to aid in release and the tableting/manufacturing process.

The immediate release portion in each of these examples comprises a mixture of the free base and hydrochloride salt of ciprofloxacin or the hydrochloride salt of ciprofloxacin alone. The API is mixed with additives to aid in release and the tableting/manufacturing process. Purified water was added and then removed during processing.

The amount of extended release portion in these bilayer tablets is between about 65% to about 67% by weight of the tablet.

A media dissolution study was performed on Example 1 and it was demonstrated that dissolution from the bilayer tablet was pH dependent.

Other dissolution studies were conducted on the formulation of Examples 1 and 4. Table 2 shows the dissolution results for coated (Examples 1 and 4) and uncoated (Example 1) bilayer tablet. These tablets were coated with Orange Opadry II^{™} (85F13899) and/or Clear Opadry^{™} (YS-1-7006) (both of which are available from ColorCon). The dissolution study was conducted in 900mL, 0.1N HCl @ 37°C +/1 0.5°C, USP Apparatus 2 (paddle) @ 50 rpm.

**Table 2:**

| | Example 1 | | Example 4 |
|---|---|---|---|
| Time (hours) | Uncoated Tablet | Coated Tablet | Coated Tablet |
| 0.25 | 41% | 41% | 42% |
| 0.5 | 48% | 47% | 56% |
| 0.75 | 54% | 52% | 69% |
| 1.0 | 60% | 57% | 86% |
| 1.5 | 74% | 68% | 99% |
| 2.0 | 87% | 84% | 100% |
| 4.0 | 100% | 102% | 100% |
| 6.0 | 100% | 102% | 100% |

Examples 5-8:

Table 3 shows extended release portions similar to those in examples 1-4. These extended release portions can be combined with immediate release portions such as, for example, those in examples 1-4. Of course, any immediate release portion can be combined with the extended release portions of examples 5-8, even immediate release portions comprising a different API.

**Table 3:**

| Ingredients | Example 5 | | Example 6 | | Example 7 | | Example 8 | |
|---|---|---|---|---|---|---|---|---|
| | mg | % | mg | % | mg | % | mg | % |
| Part I | | | | | | | | |
| Carnauba Wax | 72.9 | 8.6 | 72.9 | 8.6 | 72.9 | 8.6 | 72.9 | 8.6 |
| Stearic Acid | 24.3 | 2.9 | 24.3 | 2.9 | 24.3 | 2.9 | 24.3 | 2.9 |
| Ciprofloxacin HCl | 301.32 | 35.4 | 301.32 | 35.4 | 301.32 | 35.4 | 301.32 | 35.4 |
| Ciprofloxacin Base | 191.34 | 22.5 | 212.6 | 25.0 | 212.6 | 25.0 | 212.6 | 25.0 |

| Part II | | | | | | | | |
|---|---|---|---|---|---|---|---|---|
| Ciprofloxacin HCl | 33.48 | 3.9 | 33.48 | 3.9 | 33.48 | 3.9 | 33.48 | 3.9 |
| Ciprofloxacin Base | 21.26 | 2.5 | - | - | - | - | - | - |
| Microcrystalline Cellulose | 117.25 | 13.8 | 116.15 | 13.7 | 150.15 | 17.7 | 75.15 | 8.8 |
| Lactose, Anhydrous | - | - | - | - | - | - | 75.0 | 8.8 |
| Pregelatinized Starch | 76.5 | 9.0 | 76.5 | 9.0 | 42.5 | 5.0 | 42.5 | 5.0 |
| colloidal silocone dioxide | 3.825 | 0.5 | 4.25 | 0.5 | 4.25 | 0.5 | 4.25 | 0.5 |
| Magnesium stearate/Sodium Lauryl Sulfate (94/6) | 7.65 | 0.9 | 8.5 | 1.0 | 8.5 | 1.0 | 8.5 | 1.0 |
| Tablet Weight (mg) | 850mg | | 850mg | | 850mg | | 850mg | |

The extended release portions in examples 5-8 each contain a mixture of ciprofloxacin free base and ciprofloxacin hydrochloride salt. The API is released from a matrix comprised of carnauba wax and stearic acid. These extended release portions may be combined with an immediate release layer.

Table 4 shows the results of dissolution studies of API from the extended release portions or tablets of Examples 5-8 (no immediate release portions were combined in these dissolution studies).

**Table 4:**

| | Example 5 | Example 6 | Example 7 | Example 8 |
|---|---|---|---|---|
| 0.25 hour | 25% | 23% | 23% | 30 |
| 0.5 hour | 41% | 41% | 42% | 54 |
| 0.75 hour | 56% | 52% | 58% | 71 |
| 1.0 hour | 70% | 64% | 70% | 84 |
| 1.5 hour | 90% | 84% | 92% | 98 |
| 2.0 hour | 99% | 96% | 97% | 100 |
| 4.0 hour | 102% | - | - | 101 |
| 6.0 hour | 103% | - | - | 103 |

The dissolution conditions for examples 5-8 were as follows: 900mL, 0.1 N HC1 @ 37°C +/- 0.5°C, USP Apparatus 2 (paddle) @50 rpm.

Examples 9-11:

Examples 9 to 11 show extended release monolithic matrices comprised of ciprofloxacin free base and ciprofloxacin hydrochloride. The API is released from a matrix comprised of stearic acid, carnuba wax, and polyethylene glycol, as shown in Table 5 below.

**Table 5:**

| Ingredients | Example 9 | | Example 10 | | Example 11 | |
|---|---|---|---|---|---|---|
| | mg | % | mg | % | mg | % |
| Carnauba Wax | 40 | 4.9 | 40 | 4.9 | 40 | 4.7 |
| Stearic Acid | 40 | 4.9 | 40 | 4.9 | 40 | 4.7 |
| Polyethylene Glycol 8000 | 16 | 2.0 | 16 | 2.0 | 16 | 1.9 |
| Ciprofloxacin Hydrochloride | 334.8 | 41.3 | 334.8 | 41.3 | 334.8 | 39.2 |
| Ciprofloxacin Base | 212.6 | 26.2 | 212.6 | 26.2 | 212.6 | 24.9 |
| Microcrystalline Cellulose | 73.3 | 9.0 | 77.3 | 9.5 | 77.3 | 9.0 |
| Ethocel FP 10cps | 73.3 | 9.0 | 77.3 | 9.5 | 77.3 | 9.0 |
| Pregelatinized Starch | - | - | - | - | 45 | 5.3 |
| Colloidal Silicone Dioxide | 4.0 | 0.5 | 4.0 | 0.5 | 4.0 | 0.5 |
| Magnesium Stearate/Sodium Lauryl Sulfate (94/6) | 8.0 | 1.0 | 8.0 | 1.0 | 8.0 | .94 |
| Croscarmellose Sodium | 8.0 | 1.0 | | | | |
| Tablet weight (mg) | 810mg | | 810mg | | 855mg | |

Examples 9 through 11 can be combined in a tablet or capsule with an immediate release portion according to the present invention.

Examples 12 and 13 (Table 6) show the effect of two different levels of carnauba wax (10% vs. 5%). Examples 12 and 13 were also manufactured by hot-melt granulation. Polyethylene glycol 8000 was incorporated with carnauba wax and stearic acid to form the matrix. The dried cooled granules were screened via a #18 mesh screen, then blended with extragranular additives and compressed into tablets using a carver press.

**Table 6:**

| EXPERIMENT | Example 12 | | Example 13 | |
|---|---|---|---|---|
| | mg | % | mg | % |
| Carnauba Wax | 80.0 | 10.0 | 40.0 | 5.0 |
| Stearic Acid | 40.0 | 5.0 | 40.0 | 5.0 |
| Polyethylene Glycol 8000 | 16.0 | 2.0 | 16.0 | 2.0 |
| Polyvinypyrrolidone | 14.8 | 1.9 | 14.8 | 1.9 |
| Ciprofloxacin HCl | 334.8 | 41.8 | 334.8 | 41.8 |
| Ciprofloxacin Base | 212.6 | 26.6 | 212.6 | 26.6 |
| Dicalcium Phosphate (A-Tab) | 73.8 | 9.2 | 113.8 | 14.2 |
| Colloidal Silicone Dioxide | 4.0 | 0.5 | 4.0 | 0.5 |
| Magnesium Stearate/Sodium lauryl sulfate (94/6) | 8.0 | 1.0 | 8.0 | 1.0 |
| Croscarmellose Sodium | 16.0 | 2.0 | 16.0 | 2.0 |
| Tablet Weight (mg) | 800mg | | 800mg | |

A dissolution study for Examples 12 and 13 (see Table 7) was conducted using dissolution conditions similar to those in the examples above.

**Table 7:**

| | Example 12 | Example 13 |
|---|---|---|
| 0.25 hour | 20% | 36% |
| 0.5 hour | 33% | 57% |
| 0.75 hour | 43% | 75% |
| 1 hour | 54% | 84% |
| 1.5 hour | 70% | 89% |
| 2 hour | 84% | 91% |
| 4 hour | 93% | 91% |
| 6 hour | 94% | 91% |

Example 14 (Table 8) provides a monolithic system manufactured by granulation. In this example, ciprofloxacin HCl and ciprofloxacin base were mixed and granulated with Eudragit NE 30D^{®} (available from Rohm & Haas) suspension in a jacketed bowl. The dried cooled granules were milled by fitz-milling and blended with extragranular additives including, microcrystalline cellulose (available from FMC Bio Polymer), Dibasic Calcium Phosphate Diydrate (Emcompress), Colloidal Silicone Dioxide and Magnesium Stearate/Sodium Lauryl Sulfate (94/6) and compressed into tablets.

**Table 8:**

| | Example 14 | |
|---|---|---|
| | mg | % |
| Eudragit NE 40D | 160 | 10.8 |
| Ciprofloxacin HCl | 669.6 | 45.2 |
| Ciprofloxacin Base | 425.2 | 28.7 |
| Microcrystalline Cellulose | 101.5 | 6.9 |
| Dibasic Calcium Phosphate, Dihydrate (Emcompress) | 101.5 | 6.9 |
| Colloidal Silicone Dioxide | 7.4 | 0.5 |
| Magnesium Stearate/Sodium Lauryl Sulfate (94/6) | 14.8 | 1.0 |
| **Tablet Weight (mg)** | 1480mg | |

Examples 15 and 16 (Table 9) utilize carnauba wax and stearic acid in the extended-release portion as a matrix for API release. Succinic acid was incorporated into the matrix during granulation.

**Table 9:**

| | Example 15 | | Example 16 | |
|---|---|---|---|---|
| Extended Release Portion | mg | % | mg | % |
| Carnauba Wax | 81.0 | 9.1 | 81.0 | 9.2 |
| Stearic Acid | 27.0 | 3.03 | 27.0 | 3.1 |
| Ciprofloxacin HCl) | 334.8 | 37.6 | 334.8 | 38.0 |
| Ciprofloxacin Base | 212.6 | 23.9 | 212.6 | 24.1 |
| Succinic Acid | 45.0 | 5.05 | 30.0 | 3.40 |
| Microcrystalline Cellulose | 48.4 | 5.43 | 48.4 | 5.5 |
| Dibasic Calcium Phosphate | 48.4 | 5.43 | 48.4 | 5.5 |
| Pregelatinized Starch | 85.0 | 9.54 | 85.0 | 9.7 |
| Colloidal silicone dioxide | NA | NA | 4.3 | 0.5 |
| Magnesium Stearate/sodium lauryl sulfate (94/6) | 8.5 | 0.95 | 8.5 | 0.97 |
| Extended-release (ER) Layer Weight | 890.7 mg | | 880.0 mg | |
| Immediate Release (IR) | | | | |
| Polyvinylpyrrolidone K29/32 | 17.0 | 1.9 | 17.0 | 1.9 |
| Purified Water | (78.0) | | (78.0) | |
| Ciprofloxacin HCl, USP | 334.8 | 37.4 | 334.8 | 38.0 |
| Ciprofloxacin Base | 212.6 | 23.8 | 212.6 | 24.2 |
| Microcrystalline Cellulose | 283.85 | 31.7 | 268.85 | 32.5 |
| Croscarmellose Sodium | 34.0 | 3.8 | 17.0 | 1.9 |
| Colloidal silicone dioxide | 4.25 | 0.47 | 4.25 | 0.48 |
| Magnesium Stearate/sodium lauryl sulfate (94/6) | 8.50 | 0.95 | 8.50 | 0.96 |
| IR Layer Weight | 895.0 mg | | 880 mg | |
| Total Tablet Weight | 1785.7 mg | | 1760 mg | |

Example 17 (Table 10) utilizes carnauba wax and stearic acid in the extended-release portion as a matrix for API release. A higher amount of succinic acid was incorporated into the matrix during granulation. The amount of API in the extended-release portion of the following example was increased, compared to the examples in 15 and 16.

**Table 10:**

| | Example 17 | |
|---|---|---|
| Extended-release layer | mg | % |
| Carnauba Wax | 105.78 | 9.0 |
| Stearic Acid | 35.26 | 3.0 |
| Ciprofloxacin HCl | 437.2 | 37.4 |
| Ciprofloxacin Base | 277.64 | 23.7 |
| Succinic Acid | 60.0 | 5.13 |
| Avicel PH-200 | 63.2 | 5.40 |
| Dibasic Calcium Phosphate | 63.2 | 5.40 |
| Starch 1500 (pregelatinized starch) | 111.0 | 9.49 |
| Colloidal silicone dioxide (Cab-o-Sil, M5) | 5.62 | 0.48 |
| Magnesium Stearate/sodium lauryl sulfate (94/6) | 11.1 | 0.95 |
| Extended-release (ER) Layer Weight | 1170.0 mg | |
| Immediate Release (IR) Layer | | |
| Povidone K29/32 | 11.8 | 2.0 |
| Purified Water* | (54.14) | |
| Ciprofloxacin HCl, USP | 232.4 | 39.4 |
| Ciprofloxacin Base | 147.56 | 25.0 |
| Microcrystalline Cellulose (Avicel PH-102) | 165.78 | 28.1 |
| Ac-Di-Sol (Croscarmellose Sodium) | 23.6 | 4.0 |
| Colloidal silicone dioxide (Cab-o-Sil, M5) | 2.96 | 0.50 |
| Magnesium Stearate/sodium lauryl sulfate (94/6) | 5.9 | 1.0 |
| IR Layer Weight | 590.0 mg | |
| Total Tablet Weight | 1760 mg | |

A dissolution study for Examples 15, 16, and 17 (see Table 10) was conducted using dissolution conditions similar to the examples above.

**Table 11:**

| Time | Example 15 I | Example 16 | Example 17 |
|---|---|---|---|
| 0.25 hour | 50% | 47% | 39% |
| 0.5 hour | 64% | 62% | 50% |
| 0.75 hour | 79% | 78% | 60% |
| 1 hour | 92% | 89% | 72% |
| 1.5 hour | 98% | 97% | 93% |
| 2 hour | 98% | 98% | 98% |
| 4 hour | 100% | 99% | 99% |
| 6 hour | 101% | 101% | 100% |

Bioequivalence Study For Ciprofloxacin Extended Release Bilayer Tablets

A single-dose fasting in vivo bioequivalence study in healthy volunteers was performed comparing the ciprofloxacin extended release formulations according the present invention with CiproXR^{®} tablets from Bayer.

The fasting bioequivalence study was conducted in human subjects. The objective of this study was to investigate the bioequivalence of the ciprofloxacin extended-release 1000 mg tablets of the present invention to Bayer's Cipro^{®} XR 1000 mg tablets following a single, oral 1000 mg (1 x 1000 mg) dose administered under fasting conditions. Thirty-five (35) healthy, non-tobacco using subjects between the ages of 18 and 50 completed this open-label, single-dose, randomized, two-period, two-treatment crossover bioequivalence study. Serial blood samples (1 x 10 mL) were collected in heparinized tubes at pre-dose (within 30 minutes prior to dosing), 0.25, 0.5, 0.75, 1, 1.5, 2, 3, 4, 5, 6, 8, 10, 12, 16, 24, 36, and 48 hours post-dose. Statistical analysis of the data reveals that 90% confidence intervals are within the acceptable bioequivalent range of 80% and 125% for the natural log transformed parameters LNAUCL, LNAUCI and LNCPEAK. This study demonstrates that ciprofloxacin extended-release 1000 mg formulations according to the present invention are bioequivalent to Bayer's Cipro^{®} XR 1000 mg tablets following a single, oral 1000 mg (1 x 1000 mg) dose administered under fasting conditions.

The mean ciprofloxacin pharmacokinetic parameters in thirty-five healthy subjects following a single oral 1000 mg (1 x 1000 mg) dose of ciprofloxacin extended-release tablets (according to the present invention) under fasting conditions is shown in Table 12.

**Table 12:**

| Parameter | Arithmetic Mean A = present invention | Arithmetic Mean B = Cipro^{®} XR |
|---|---|---|
| AUCL (µg x hr/mL) | 22.75 | 24.73 |
| AUCI (µg x hr/mL) | 23.35 | 25.41 |
| CPEAK (µg/mL) | 3.902 | 4.227 |
| TPEAK (hr) | 2.600 | 2.386 |

A bioequivalence study was also conducted under fed conditions in healthy human subjects. The objective of this study was to investigate the bioequivalence of the ciprofloxacin extended-release formulations of the present invention to Bayer's Cipro^{®} XR 1000 mg tablets following a single, oral 1000 mg (1 x 1000 mg) dose administered under fed conditions. Thirty-four (34) healthy, non-tobacco using subjects between the ages of 18 and 56 completed this open-label, single-dose, randomized, two-period, two-treatment crossover bioequivalence study was conducted. Serial blood samples (1 x 10 mL) were collected in heparinized tubes at pre-dose (within 45 minutes prior to dosing), 0.25, 0.5, 0.75, 1, 1.5, 2, 3, 4, 5, 6, 8, 10, 12, 16, 24, 36, and 48 hours post-dose. Plasma samples were stored at -70°C ± 15°C until shipped for analysis. Statistical analysis of the data reveals that 90% confidence intervals are within the acceptable bioequivalent range of 80% and 125% for the natural log transformed parameters LNAUCL, LNAUCI and LNCPEAK. This study demonstrates that ciprofloxacin extended-release 1000 mg tablets according to the present invention are bioequivalent to Bayer's Cipro^{®} XR 1000 mg tablets following a single, oral 1000 mg (1 x 1000 mg) dose administered under fed conditions.

The mean ciprofloxacin pharmacokinetic parameters in thirty-five healthy subjects following a single oral 1000 mg (1 x 1000 mg) dose of the ciprofloxacin extended-release formulation according to the present invention under fed conditions is shown in Table 13.

**Table 13:**

| Parameter | Arithmetic Mean A = present invention | Arithmetic Mean B = Cipro^{®} XR |
|---|---|---|
| AUCL (µg x hr/mL) | 21.26 | 21.25 |
| AUCI (µg x hr/mL) | 22.04 | 22.12 |
| CPEAK (µg/mL) | 3.439 | 3.352 |
| TPEAK (hr) | 3.706 | 2.926 |

A 500 mg formulation was manufactured, dosed, and compositionally proportional to the 1000 mg formulations described above. The bioavailabity study was conducted for 500mg in fasting conditions in thirty three healthy subjects. This study demonstrates that ciprofloxacin extended-release 500 mg tablets according to the present invention are bioequivalent to Bayer's Cipro^{®} XR 500 mg tablets following a single, oral 500 mg (1 x 500 mg) dose administered under fasting conditions. The mean ciprofloxacin pharmacokinetic parameters in thirty-four healthy subjects following a single oral 500 mg (1 x 500 mg) dose of the ciprofloxacin extended-release formulation according to the present invention under fasting conditions is shown in Table 14.

**Table 14:**

| Parameter | Arithmetic Mean A=present invention | Arithmetic Mean B = Cipro^{®} XR |
|---|---|---|
| AUCL(µg x hr/mL) | 10.95 | 11.53 |
| AUCI(µg x hr/mL) | 11.43 | 12.06 |
| CPEAK (µg/mL) | 2.252 | 2.211 |
| TPEAK (hr) | 1.864 | 1.720 |

It is believed that when the API is ciprofloxacin, a formulation according to the present invention provides for dissolution profiles which are similar to those achieved with Bayer's CiproXR^{®} product. As such, some of the formulations according to the present invention, when the API is ciprofloxacin, may be considered "bioequivalent" to CiproXR^{®}. However, this application is not limited to formulations comprising ciprofloxacin..

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.
Aspects of the present invention are defined by way of the following clauses:
1. An extended release pharmaceutical formulation comprising an extended release portion and an immediate release portion, said extended release portion comprising:
   (a) an active pharmaceutical ingredient (API), and (b) a wax in an amount of about 2% to about 40% by weight of said extended release portion.
2. The extended release pharmaceutical formulation of clause 1, wherein said amount of wax is about 4% to about 30% by weight of said extended release portion.
3. The extended release pharmaceutical formulation of clause 1, wherein said amount of wax is about 6% to about 23% by weight of said extended release portion.
4. The extended release pharmaceutical formulation of clause 1, wherein said active pharmaceutical ingredient is selected from the group consisting of propranolol, metoprolol, metoprolol tartrate, galantamine, bupropion, diltiazem, oxybutynin, hydrochlorothiazide, metformin, opamine, ciprofloxacin, vancomycin, norvancomycin, daunorubicin, vinca alkaloids, cetrizine, venlafaxine, opioid analgesics, theophylline, verapamil, amlodipine, tramadol, diltiazem, timolol, trospium, pramipexole, and pharmaceutically acceptable salts, hydrates, and solvates thereof.
5. The extended release pharmaceutical formulation of clause 1, wherein an amount of said API in said extended release portion ranges from about 5% to about 75% by weight of said extended release portion.
6. The extended release pharmaceutical formulation of clause 1, wherein an amount of said API in said extended release portion ranges from about 30% to about 70% by weight of said extended release portion.
7. The extended release pharmaceutical formulation of clause 1, wherein an amount of said API in said extended release portion ranges from about 55% to about 60% by weight of said extended release portion.
8. The extended release pharmaceutical formulation of clause 1, wherein said active pharmaceutical ingredient is a mixture of a free base and a salt.
9. The extended release pharmaceutical formulation of clause 8, wherein the ratio of said free base to said salt is about 1:2 to about 2:1.
10. The extended release pharmaceutical formulation of clause 8, wherein said API is ciprofloxacin.
11. The extended release pharmaceutical formulation of clause 1, wherein said extended release portion further comprises a matrix-forming component selected from the group consisting of succinic acid, citric acid, malic acid, stearic acid, succinic acid, lactic acid, aspartic acid, glutamic acid, gluconic acid, acetic acid, formic acid, hydrochloric acid, sulphuric acid, phosphoric acid, hydrophilic polymers, polyethylene glycols, pH dependent acrylate polymers or copolymers, and pore forming agents.
12. The extended release pharmaceutical formulation of clause 1, wherein said extended release portion further comprises dibasic calcium phosphate in an amount of about 3% to about 30% by weight of said extended release portion.
13. The extended release pharmaceutical formulation of clause 1, wherein said immediate release portion comprises ciprofloxacin.
14. The extended release pharmaceutical formulation of clause 1, wherein the ratio of said extended release portion to said immediate release portion in said formulation is from about 9:1 or about 1:9.
15. An extended release pharmaceutical composition comprising an extended release portion and an immediate release portion, said extended release portion comprising a wax and ciprofloxacin.
16. The extended release pharmaceutical formulation of clause 15, wherein an amount of said wax in said extended release portion ranges from about 2% to about 40% by weight of said extended release portion.
17. The extended release pharmaceutical formulation of clause 16, wherein said amount is from about 4% to about 30% by weight of said composition.
18. The extended release pharmaceutical formulation of clause 17, wherein said amount is from about 6% to about 23% by weight of said composition.
19. The extended release pharmaceutical formulation of clause 15, wherein an amount of said active pharmaceutical ingredient in said extended release portion is from about 55% to about 60% by weight of the extended release portion.
20. The extended release pharmaceutical formulation of clause 15, wherein said immediate release portion comprises an active pharmaceutical ingredient selected from the group consisting of propranolol, metoprolol, metoprolol tartrate, galantamine, bupropion, diltiazem, oxybutynin, hydrochlorothiazide, metformin, opamine, ciprofloxacin, vancomycin, norvancomycin, daunorubicin, vinca alkaloids, cetrizine, venlafaxine, opioid analgesics, theophylline, verapamil, amlodipine, tramadol, diltiazem, timolol, trospium, pramipexole, and pharmaceutically acceptable salts, hydrates, and solvates thereof.
21. An extended release pharmaceutical formulation comprising:
   a. an extended release portion comprising an API and carnauba wax; and
   b. an immediate release portion comprising an API;
      said extended and immediate release portions both comprising the same API.
22. The extended release pharmaceutical formulation of clause 21, wherein said API is ciprofloxacin.
23. The extended release pharmaceutical formulation of clause 21, wherein an amount of said carnauba wax is from about 2% to about 40% by weight of said composition.
24. The extended release pharmaceutical formulation of clause 23, wherein said amount of said carnauba wax is from about 4% to about 30% by weight of said composition.
25. An extended release pharmaceutical formulation comprising:
   a. an extended release portion comprising ciprofloxacin in an amount of from about 30% to about 70% by weight of said extended release portion; a wax in the amount of about 6% to about 23% by weight of the extended release portion; and stearic acid; and
   b. an immediate release portion comprising ciprofloxacin in an amount of from about 5% to about 80% by weight of said immediate release portion.
26. The extended release pharmaceutical formulation of clause 25, wherein said ciprofloxacin in said extended and immediate release portions is a mixture of a ciprofloxacin free base and a ciprofloxacin salt.
27. The extended release pharmaceutical formulation of clause 25, wherein said extended and immediate release portions are comprised of particles, wherein a majority of said particles have a size of at least about 40 mesh.
28. The extended release pharmaceutical formulation of clause 25, wherein about 0% to about 20% of said API is released from said formulation after about 0.25 hours; wherein about 15% to about 35% of said API is released from said formulation after about 0.5 hours; wherein about 35% to about 50% of said API is released from said formulation after about 1 hour; wherein about 50% to about 75% of said API is released from said formulation after about 2 hours; wherein about 60% to about 85% of said API is released from said formulation after about 6 hours; and wherein at least about 80% of said API is released from said formulation after about 8 hours.
29. The extended release pharmaceutical formulation of clause 25, wherein said amount of API in said extended release portion is from about 55% to about 65%.

## Claims

1. An extended release pharmaceutical formulation comprising an extended release portion and an immediate release portion, said extended release portion comprising: (a) an active pharmaceutical ingredient (API), and (b) a wax in an amount of about 2% to about 40% by weight of said extended release portion.

2. The extended release pharmaceutical formulation of claim 1, wherein said amount of wax is about 4% to about 30% by weight of said extended release portion.

3. The extended release pharmaceutical formulation of claim 1, wherein said amount of wax is about 6% to about 23% by weight of said extended release portion.

4. The extended release pharmaceutical formulation of claim 1, wherein said active pharmaceutical ingredient is selected from the group consisting of propranolol, metoprolol, metoprolol tartrate, galantamine, bupropion, diltiazem, oxybutynin, hydrochlorothiazide, metformin, opamine, ciprofloxacin, vancomycin, norvancomycin, daunorubicin, vinca alkaloids, cetrizine, venlafaxine, opioid analgesics, theophylline, verapamil, amlodipine, tramadol, diltiazem, timolol, trospium, pramipexole, and pharmaceutically acceptable salts, hydrates, and solvates thereof.

5. The extended release pharmaceutical formulation of claim 1, wherein an amount of said API in said extended release portion ranges from about 5% to about 75% by weight of said extended release portion.

6. The extended release pharmaceutical formulation of claim 1, wherein an amount of said API in said extended release portion ranges from about 30% to about 70% by weight of said extended release portion.

7. The extended release pharmaceutical formulation of claim 1, wherein an amount of said API in said extended release portion ranges from about 55% to about 60% by weight of said extended release portion.

8. The extended release pharmaceutical formulation of claim 1, wherein said active pharmaceutical ingredient is a mixture of a free base and a salt.

9. The extended release pharmaceutical formulation of claim 8, wherein the ratio of said free base to said salt is about 1:2 to about 2: 1.

10. The extended release pharmaceutical formulation of claim 8, wherein said API is ciprofloxacin.

11. The extended release pharmaceutical formulation of claim 1, wherein said extended release portion further comprises a matrix-forming component selected from the group consisting of succinic acid, citric acid, malic acid, stearic acid, succinic acid, lactic acid, aspartic acid, glutamic acid, gluconic acid, acetic acid, formic acid, hydrochloric acid, sulphuric acid, phosphoric acid, hydrophilic polymers, polyethylene glycols, pH dependent acrylate polymers or copolymers, and pore forming agents.

12. The extended release pharmaceutical formulation of claim 1, wherein said extended release portion further comprises dibasic calcium phosphate in an amount of about 3% to about 30% by weight of said extended release portion.

13. The extended release pharmaceutical formulation of claim 1, wherein said immediate release portion comprises ciprofloxacin.

14. The extended release pharmaceutical formulation of claim 1, wherein the ratio of said extended release portion to said immediate release portion in said formulation is from about 9:1 or about 1:9.

15. An extended release pharmaceutical formulation comprising:
a. an extended release portion comprising an API and carnauba wax; and
b. an immediate release portion comprising an API;
said extended and immediate release portions both comprising the same API.

16. The extended release pharmaceutical formulation of claim 15, wherein said API is ciprofloxacin.

17. The extended release pharmaceutical formulation of claim 15, wherein an amount of said carnauba wax is from about 2% to about 40% by weight of said composition.

18. The extended release pharmaceutical formulation of claim 17, wherein said amount of said carnauba wax is from about 4% to about 30% by weight of said composition.

19. An extended release pharmaceutical formulation comprising:
a. an extended release portion comprising ciprofloxacin in an amount of from about 30% to about 70% by weight of said extended release portion; a wax in the amount of about 6% to about 23% by weight of the extended release portion; and stearic acid; and
b. an immediate release portion comprising ciprofloxacin in an amount of from about 5% to about 80% by weight of said immediate release portion.

20. The extended release pharmaceutical formulation of claim 19, wherein said ciprofloxacin in said extended and immediate release portions is a mixture of a ciprofloxacin free base and a ciprofloxacin salt.

21. The extended release pharmaceutical formulation of claim 19, wherein said extended and immediate release portions are comprised of particles, wherein a majority of said particles have a size of at least about 40 mesh.

22. The extended release pharmaceutical formulation of claim 19, wherein about 0% to about 20% of said API is released from said formulation after about 0.25 hours; wherein about 15% to about 35% of said API is released from said formulation after about 0.5 hours; wherein about 35% to about 50% of said API is released from said formulation after about 1 hour; wherein about 50% to about 75% of said API is released from said formulation after about 2 hours,- wherein about 60% to about 85% of said API is released from said formulation after about 6 hours; and wherein at least about 80% of said API is released from said formulation after about 8 hours.

23. The extended release pharmaceutical formulation of claim 19, wherein said amount of API in said extended release portion is from about 55% to about 65%.
